**Europäisches Patentamt**

**European Patent Office**

**Office européen des brevets**

(19)

(11) Veröffentlichungsnummer: **0 223 035**
**B1**

(12) **EUROPÄISCHE PATENTSCHRIFT**

(45) Veröffentlichungstag der Patentschrift:
15.11.89

(51) Int. Cl.⁴: **C07C 85/11**, C07C 87/58,
B01J 25/00, B01J 25/02

(21) Anmeldenummer: **86113764.4**

(22) Anmeldetag: **04.10.86**

(54) **Verwendung von modifizierten Raney-Katalysatoren zur Herstellung von aromatischen Diaminoverbindungen.**

(30) Priorität: **19.10.85 DE 3537247**

(43) Veröffentlichungstag der Anmeldung:
**27.05.87 Patentblatt 87/22**

(45) Bekanntmachung des Hinweises auf die Patenterteilung:
**15.11.89 Patentblatt 89/46**

(84) Benannte Vertragsstaaten:
**BE DE ES FR GB IT NL**

(56) Entgegenhaltungen:
**EP-A- 0 091 027**
**DE-A- 2 713 374**
**FR-A- 1 066 582**
**GB-A- 919 273**

**PATENT ABSTRACTS OF JAPAN, Band 4,
Nr. 69 (C-11)[551], 22. Mai 1980; & JP - A - 55 35064**

(73) Patentinhaber: **BAYER AG,
D-5090 Leverkusen 1 Bayerwerk(DE)**

(72) Erfinder: **Becher, Dieter, Dr., Moltkestrasse 8,
D-4047 Dormagen(DE)**
Erfinder: **Birkenstock, Udo, Dr., Schneppersdelle 2,
D-4030 Ratingen 8(DE)**
Erfinder: **Waldau, Eckart, Dr., Benrather Schlossufer 7,
D-4000 Düsseldorf 13(DE)**
Erfinder: **Witt, Harro, Dr., Möhlenbag 2,
D-2224 Kuden(DE)**

**Beschreibung**

Die Erfindung betrifft die Verwendung von modifizierten Raney-Katalysatoren zur Herstellung von aromatischen Diaminoverbindungen durch Hydrierung von aromatischen Dinitroverbindungen.

Die katalytische Hydrierung aromatischer Dinitroverbindungen wie z.B. dem Dinitrotoluol an suspendierten Raney-Katalysatoren ist an sich bekannt (vergl. z.B. DE-AS 1 044 099 oder FR-PS 1 599 004).

Ihre technische Ausführung erfolgt zumeist bei Temperaturen unterhalb 170°C, vorzugsweise bei Temperaturen zwischen 50 und 150°C. Dabei wird die Reaktionswärme der Hydrierung bewußt verloren gegeben, um die gewünschten Diamine in hohen Ausbeuten und hohen Reinheiten erhalten zu können. So beschreibt beispielsweise die EP-A 0 091 027 spezielle Raney-Trägerkatalysatoren, die außer zur Hydrierung von aromatischen zu den entsprechenden cycloaliphatischen Verbindungen auch zur Überführung von Nitroverbindungen in die entsprechenden Aminoverbindungen geeignet sind. Bei dieser Hydrierung wird jedoch die Einhaltung von Temperaturen von 50–100°C empfohlen. Verfahren andererseits, die eine Reaktionsführung bei hohen Temperaturen gestatten – wie z.B. in DE-OS 3 315 191 beschrieben – bedürfen verbesserter Katalysatoren mit erhöhter Selektivität und erhöhter Temperaturbeständigkeit. Wichtige Nebenreaktionen sind in diesem Zusammenhang Hydrierungen und Desaminierungen der aromatischen Kerne, sowie die Bildung von höhermolekularen Verbindungen. Auch dies wird durch die Lehre der EP-A 0 091 027 bestätigt, wo darauf aufmerksam gemacht wird, daß bei einer Erhöhung der Hydriertemperatur auf oberhalb 175°C eine Kernhydrierung stattfindet, so daß davon ausgegangen werden muß, daß die in dieser Veröffentlichung beschriebene Verfahrensweise zur Herstellung von aromatischen Diaminoverbindungen durch Hydrierung bei vergleichsweise hohen Temperaturen ungeeignet ist. Auch gemäß der Lehre der DE-OS 2 713 374 erfolgt die Hydrierung von aromatischen Nitroverbindungen wie Dinitrotoluol zu den entsprechenden Diaminen unter Verwendung der Raney-Nickel-Eisen-Katalysatoren dieser Vorveröffentlichung bei unter 170°C liegenden Temperaturen, wobei zudem Hilfslösungsmittel mitverwendet werden.

Ziel der nachfolgend beschriebenen Verwendung von modifizierten Raney-Katalysatoren zur Herstellung von aromatischen Diaminoverbindungen ist es, Reaktionsbedingungen zu ermöglichen, die es gestatten, die Reaktionswärme der Hydrierung in einer technisch interessanten Form – z.B. als Dampf – zu gewinnen, ohne Einbußen bei Produktqualität und Ausbeuten hinzunehmen, wobei zwecks Vereinfachung der Hydrierung diese vorzugsweise in Abwesenheit von Hilfslösungsmitteln erfolgen sollte. Dabei wird der wirtschaftliche Nutzen um so höher ausfallen, je höher die Reaktionstemperatur ohne Ausbeuteverluste gewählt werden kann. Der aus der Reaktionswärme des Verfahrens gewonnene Dampf kann im Zuge der Aufarbeitung des Diamins genutzt oder an andere Energieverbraucher abgegeben werden.

Es wurde gefunden, daß die beschriebenen Begrenzungen der großtechnisch ausgeübten Verfahren dadurch überwunden werden können, daß neue, verbesserte Katalysatoren eingesetzt werden.

Gegenstand der Erfindung ist somit die Verwendung von modifizierten Raney-Katalysatoren bei der Herstellung von aromatischen Diaminoverbindungen durch Hydrierung der entsprechenden aromatischen Dinitroverbindung, dadurch gekennzeichnet, daß man die Umsetzung der Temperaturen von 170–250°C und bei Drucken von 15–50 bar in Abwesenheit eines Hilfslösungsmittels durchführt und als Raney-Katalysatoren solche verwendet, die durch Alkalibehandlung einer Legierung aus 50 bis 95 Gew.-%, vorzugsweise 60 bis 85 Gew.-% Aluminium, 4 bis 45 Gew.-%, vorzugsweise 10 bis 30 Gew.-% Nickel und/oder Kobalt und 1 bis 46 Gew.-%, vorzugsweise 5 bis 15 Gew.-% mindestens eines modifizierenden Metalls hergestellt worden sind, wobei sich die genannten Prozentsätze zu 100 ergänzen, und wobei die modifizierenden Metalle solche der 1., 4., 5., 6., 7. und/oder 8. Nebengruppe des Periodensystems der Elemente sind.

Bei der erfindungsgemäßen Verwendung werden die zuletzt genannten Raney-Katalysatoren eingesetzt. Vorzugsweise handelt es sich um solche Raney-Katalysatoren der genannten Art, in denen Eisen, Ruthenium, Rhenium, Chrom, Molybdän, Wolfram, Niob, Tantal, Vanadin, Titan, Kupfer, Zirkonium und/oder Hafnium als modifizierende Metalle vorliegen.

Die Alkalibehandlung kann beispielsweise dergestalt erfolgen, daß man die Legierungen mit wäßrigen Lösungen von Alkalihydroxiden oder, weniger bevorzugt, -carbonaten, beispielsweise von Natrium- oder Kaliumhydroxid oder, weniger bevorzugt, von Natrium- oder Kaliumcarbonat, vorzugsweise bei erhöhten Temperaturen von ca. 50 - 100° C zur Reaktion bringt, den Katalysator abfiltriert und durch Waschen mit Wasser (z.B. destilliertes, entionisiertes und/oder Trinkwasser) von der Hauptmenge der alkalischen Bestandteile befreit. Die Menge der Basen richtet sich hierbei nach der Menge des in der Legierung vorliegenden Aluminiums. Die Basen können in einer Menge eingesetzt werden, die bezüglich der Überführung der Gesamtmenge des Aluminiums in Alkalialuminat unterstöchiometrisch oder stöchiometrisch ist; vorzugsweise werden die Alkaliverbindungen jedoch in einem bezüglich der Aluminatsbildung stöchiometrischen Überschuß eingesetzt.

Die so erhaltenen Katalysatoren können noch Restmengen an Aluminium aufweisen. So liegt diese Restmenge an Aluminium im allgemeinen bei 1 bis 70, vorzugsweise 1 bis 10 Gew.-%. Das Gewichtsverhältnis von Nickel und/oder Kobalt zu den modifizierenden Metallen liegt in den Katalysatoren im allgemeinen bei 30:70 bis 99:1, vorzugsweise bei 50:50 bis 90:10.

Die erfindungswesentlichen Katalysatoren stellen modifizierte Skelett-Katalysatoren vom Typ Raney dar und liegen im allgemeinen in Form von Feuchtpulvern einer mittleren Teilchengröße von <130 μm, vorzugsweise von <80 μm vor, die mit einem Laser Granulometer in an sich bekannter Weise bestimmbar ist.

Änliche Katalysatoren sind in den japanischen Patentanmeldungen 54 084 - 508 vom 15.12.77, veröffentlicht am 5.7.79 unter der Nr. 152 098 und 55 035 - 064 vom 5.9.78, veröffentlicht am 11.3.1980 unter der Nummer 109 240 für die Reduktion von Aldehyden beschrieben, ihre gute Eignung für die Hydrierung von aromatischen Dinitroverbindungen war jedoch, wegen des unterschiedlichen Reaktionsverlaufs, der unterschiedlichen Reaktionsbedingungen und der für diesen Fall nicht relevanten Nutzungsmöglichkeit der Reaktionswärme weder naheliegend noch im Ergebnis vorhersehbar.

Ausgangsmaterialien sind beliebige aromatische Dinitroverbindungen, z.B. 1,3-Dinitrobenzol, 1,5-Dinitronaphthalin, 1,8-Dinitronaphthalin oder Isomere des Dinitrotoluols bzw. deren Gemische. Die erfindungsgemäß zu verwendenden Katalysatoren eignen sich insbesondere zur katalytischen Hydrierung des 2,4-Dinitrotoluols bzw. seiner technischen Gemische mit bis zu 40 Gew.-%, bezogen auf Gesamtgemisch, an 2,6-Dinitrotoluol.

Die Dinitroverbindungen werden in Substanz, d.h. ohne Mitverwendung eines inerten Lösungsmittels bei einer Temperatur von 170 bis 250°C und einem Druck von 15 bis 50 bar (ohne Lösungsmittel) bzw. 15 bis 120 bar (mit Lösungsmittel) in Gegenwart der in dem Reaktionsmedium dispergierten Katalysatoren hydriert. Bei Raumtemperatur feste Dinitroverbindungen werden hierbei vor der Durchführung der Hydrierung aufgeschmolzen.

Die Hydrierung kann sowohl diskontinuierlich als auch kontinuierlich unter Verwendung der üblichen Reaktoren erfolgen. Beispielsweise kann die Hydrierung kontinuierlich in einem Reaktor der in Beispiel 2 beschriebenen Art durchgeführt werden, der vor der Beschickung mit der zu hydrierenden Dinitroverbindung mit einer Suspension des Katalysators in Verfahrensprodukt (Gemisch aus Diamin und Wasser) gefüllt worden ist. Bei der kontinuierlichen Durchführung in einem derartigen Reaktor entspricht die Menge der in den Reaktor eingespeisten Dinitroverbindung der gleichzeitig entnommenen Menge an Verfahrensprodukt. Die sich aus dem Durchsatz der aromatischen Dinitroverbindung, der Art und Menge des eingesetzten Katalysators und dem Reaktorvolumen ergebende mittlere Verweilzeit des Reaktionsgemischs im Reaktor liegt im allgemeinen zwischen einer Minute und drei Stunden.

Die Reaktionswärme kann wegen der vergleichsweisen hohen Reaktionstemperatur in an sich bekannter Weise zur Erzeugung von Dampf mit Abgabedrucken zwischen 5 und 30 bar genutzt werden.

Die nachfolgenden Beispiele dienen zur weiteren Erläuterung der Erfindung, ohne diese zu begrenzen. Alle Prozentangaben beziehen sich auf Gewichtsprozente.

Beispiel 1 (Herstellung eines Katalysators)

782 g Natriumhydroxid werden in einem Becherglas in 3129 g Wasser gelöst. Die Temperatur der Natriumhydroxidlösung wird auf 80° C eingestellt. Die Luft über der Lösung wird durch Stickstoff ausgetauscht, damit während der gesamten Reaktionszeit unter einem Stickstoffschleier gearbeitet werden kann.

Anschließend werden insgesamt 200 g einer Legierung aus 80 Teilen Aluminium, 14 Teilen Nickel und 6 Teilen Eisen zu der Natriumhydroxid-Lösung in Portionen zu jeweils 6 g hinzugefügt. Es entsteht jeweils eine stark exotherme Reaktion mit heftiger Schaumbildung. Die Zugabe der Legierung erfolgt über einen Zeitraum von 20 min., so daß die Temperatur der Natriumhydroxid-Lösung bei 80° C ± 2° C gehalten wird und die Schaumbildung nicht zu stark ansteigt.

Nachdem die Gesamtmenge der Legierung eingetragen ist, rührt man das Reaktionsgemisch während 30 min. bei 80° C. Anschließend wird die Mutterlauge vom Katalysator abdekantiert und der Katalysator mit einer Waschlauge, bestehend aus 78 g Natriumhydroxid und 313 g Wasser während 5 min. unter Rühren nachbehandelt. Schließlich wird die Waschlauge durch Dekantieren abgetrennt und der Katalysator mit Wasser bis zu einem pH-Wert von ca. 8,0 gewaschen. Es werden 105 g Katalysatorschlamm erhalten. Das Gewichtsverhältnis von Nickel zu Eisen liegt bei 69 : 31.

Beispiel 2 (erfindungsgemäße Verwendung)

Zum Einsatz gelangt ein Autoklav mit 80 ml Flüssigvolumen, versehen mit einem Begasungsrührer, einer Wasserstoffzuführungsleitung, einem Einleitungsrohr für die aromatische Dinitroverbindung, dessen unteres Ende direkt am Begasungsrührer endet, und einem Auslaßventil für überschüssigen Wasserstoff. Das Reaktionsgemisch aus einem aromatischen Diamin und Wasser verläßt den Reaktor durch eine Fritte, die den Katalysator zurückhält. Die Apparatur ermöglicht die kontinuierliche Hydrierung der aromatischen Dinitroverbindung bis zur völligen Erschöpfung des Katalysators. Die Temperatur im Reaktor wird durch einen äußeren Heiz- bzw. Kühlkreislauf geregelt, eine Kühlschlange im Inneren des Reaktors kann für zusätzliche Kühlung des Reaktionsgemisches sorgen.

80 ml eines Gemischs aus (i) einem Gemisch aus 80 % 2,4-Diaminotoluol und 20 % 2,6-Diaminotoluol (TDA) mit (ii) Wasser im Gewichtsverhältnis (i):(ii) 63 : 37, in welchem 1,6 g des Katalysators gemäß Beispiel 1 suspendiert vorliegen, werden in dem Reaktor vorgelegt. Anschließend wird der Reaktorinhalt unter einer Wasserstoffatmosphäre bei einem Druck von 20 bar auf 175° C aufgeheizt. Bei dieser Tempe-

ratur werden stündlich 64 l Wasserstoff und mittels einer Dosierpumpe 53 g DNT (Gemisch aus 80 % 2,4-Dinitrotoluol und 20 % 2,6-Dinitrotoluol) in den Reaktor geleitet, wobei die Temperatur im Reaktor auf 190° C steigt. Bei dieser Temperatur und einem Druck von 20 bar wird bis zur Erschöpfung des Katalysators (50 h) hydriert, wobei konti nuierlich das resultierende Verfahrensprodukt (Gemisch aus Diamin und Wasser) entnommen wird, aus welchem anschließend destillativ das reine Diaminotoluol Isomerengemisch (TDA) gewonnen wird. Die Ausbeute an reinem TDA (80 : 20) entspricht 98,7 % der Theorie, bezogen auf eingesetztes DNT. Bei der destillativen Aufarbeitung fallen 1,1 % teerartige Nebenprodukte und 0,2 % "Niedrigsieder" an.

Beispeil 3 (erfindungsgemäße Verwendung)

Als Katalysator wird in diesem Beispiel ein modifiziertes Raney-Nickel verwendet, welches entsprechend Beispiel 1 durch Alkalibehandlung einer Legierung aus 80 % Aluminium, 10 % Nickel und 10 % Molybdän erhalten worden ist. Das Gewichtsverhältnis Nickel : Molybdän im Katalysator liegt bei 74 : 26.

Es wird in der im Beispiel 2 beschriebenen Apparatur bei einer Temperatur von 220° C und einem Druck von 30 bar gearbeitet, wobei entsprechend Beispiel 2 53 g/h DNT zum Einsatz gelangen und die Befüllung des Reaktors vor Beginn der Reaktion (Diamin und Wasser) 0,8 g Katalysator suspendiert enthält. Die Ausbeute an TDA entspricht 99,2 % der Theorie, bezogen auf eingesetztes DNT. Daneben bilden sich 0,75 % teerartige Produkte und 0,05 % tiefer als TDA siedende Nebenprodukte ("Niedrigsieder").

Beispiele 4 - 14 (erfindungsgemäße Verwendung)

Beispiel 2 wird wiederholt unter Verwendung der nachstehend tabellarisch zusammengestellten Katalysatoren, Temperaturen und Drücke. Die Herstellung der Katalysatoren erfolgte jeweils in Analogie zu Beispiel 1 unter Verwendung der, bezogen auf den Aluminiumgehalt der Legierung, aliquoten Menge an Natronlauge. Die in den Formeln hinter den Elementen angegebenen Zahlen beziehen sich auf die prozentuale Zusammensetzung der Legierungen bzw. auf die Gewichtsverhältnisse des in den Katalysatoren vorliegenden Nickels bzw. modifizierenden Metalls. Die Katalysatoren enthalten noch schwankende Restmenge an Aluminium (1 - 10 %). In Beispiel 13 wird unter Mitverwendung von Isopropanol als Lösungsmittel gearbeitet. Dies bedeutet, daß zur Befüllung des Reaktors vor Beginn der Reaktion eine 30-gew.-%ige Lösung von TDA/Wasser (63 : 37), in welcher der Katalysator suspendiert vorliegt, verwendet wird, und das zu hydrierende DNT als 33-gew.-%ige Lösung in Isopropanol eingesetzt wird. Die Gesamtmenge der kontinuierlich eingespeisten Lösung entspricht hierbei einem Einsatz von 126 g/h DNT. Die Ergebnisse der Beispiele 4 - 14 sind in nachstehender Tabelle zusammengefaßt.

**Tabelle**

| Beispiel | Legierung | Katalysator | t (°C) | p (bar) | TDA-Ausbeute (%) |
|---|---|---|---|---|---|
| 4 | Al80Ni15Nb5 | RaNi81Nb19 | 220 | 30 | 97,5 |
| 5 | Al85Ni14Ta1 | RaNi94Ta6 | 220 | 30 | 98 |
| 6 | Al80Ni14W6 | RaNi93W7 | 220 | 30 | 97 |
| 7 | Al70Ni25,5Ti4,5 | RaNi86Ti14 | 190 | 30 | 98,2 |
| 8 | Al70Ni25,5V4,5 | RaNi98V2 | 190 | 30 | 98,7 |
| 9 | Al80Ni14Cr6 | RaNi77Cr23 | 190 | 30 | 97,5 |
| 10 | Al80Ni14Cu6 | RaNi70Cu30 | 220 | 30 | 98,9 |
| 11 | Al80Ni14Mo6 | RaNi88Mo12 | 220 | 30 | 99 |
| 12 | Al80Ni12Fe6Mo2 | RaNi64Fe32Mo4 | 190 | 30 | 99 |
| 13 | Al90Ni7Fe3 | RaNi75Fe25 | 190 | 30 | 98,7 |

**Patentansprüche**

1. Verwendung von modifizierten Raney-Katalysatoren bei der Herstellung von aromatischen Diaminoverbindungen durch Hydrierung der entsprechenden aromatischen Dinitroverbindung, dadurch gekennzeichnet, daß man die Umsetzung bei Temperaturen von 170–250°C und bei Drucken von 15–50 bar in Abwesenheit eines Hilfslösungsmittels durchführt und als Raney-Katalysatoren solche verwendet, die durch Alkalibehandlung einer Legierung aus 50 bis 95 Gew.-% Aluminium, 4 bis 45 Gew.-% Nickel und/oder Kobalt und 1 bis 46 Gew.-% mindestens eines modifizierenden Metalls hergestellt worden sind, wobei sich die genannten Prozentsätze zu 100 ergänzen, und wobei die modifizierenden Metalle solche der 1., 4., 5., 6., 7. und/oder 8. Nebengruppe des Periodensystems der Elemente sind.

2. Verwendung gemäß Anspruch 1, dadurch gekennzeichnet, daß das modifizierende Metall ausgewählt ist aus der Gruppe bestehend aus Eisen, Chrom, Kupfer, Molybdän, Tantal, Wolfram, Vanadin, Titan, Niob, Rhenium, Ruthenium, Zirkonium und Hafnium.

3. Verwendung gemäß Anspruch 1, dadurch gekennzeichnet, daß man die Reaktionswärme der Umsetzung zur Erzeugung von Dampf mit Abgabedrucken zwischen 5 und 30 bar nutzt.

## Claims

1. The use of modified Raney catalysts in the production of aromatic diamino compounds by hydrogenation of the corresponding aromatic dinitro compound, characterized in that the reaction is carried out at temperatures in the range from 170 to 250°C and under pressure of from 15 to 50 bar in the absence of an auxiliary solvent and the Raney catalysts used are those which have been obtained by alkali treatment of an alloy of 50 to 95% by weight aluminium, 4 to 45% by weight nickel and/or cobalt and 1 to 46% by weight of at least one modifying metal, the percentages mentioned adding up to 100 and the modifying metals being those of the 1st, 4th, 5th, 6th, 7th and/or 8th secondary group of the periodic system of elements.

2. The use claimed in claim 1, characterized in that the modifying metal is selected from the group consisting of iron, chromium, copper, molybdenum, tantalum, tungsten, vanadium, titanium, niobium, rhenium, ruthenium, zirconium and hafnium.

3. The use claimed in claim 1, characterized in that the heat of the reaction is used to produce steam with delivery pressures of from 5 to 30 bar.

## Revendications

1. Utilisation de catalyseurs Raney modifiés dans la fabrication de composés diamino aromatiques par hydrogénation des composés dinitro aromatiques correspondants, caractérisée en ce qu'on exécute la réaction à des températures de 170 à 250°C et à des pressions de 15 à 50 bar en l'absence d'un solvant auxiliaire et en ce qu'on utilise, comme catalyseurs Raney, des catalyseurs fabriqués par traitement aux alcalis d'un alliage composé de 50 à 95% en poids d'aluminium, 4 à 45% en poids de nickel et/ou de cobalt et 1 à 46% en poids au moins d'un métal modifiant, le total des pourcentages indiqués devant être 100, et les métaux modifiants appartenant au 1er, 4e, 5e, 6e, 7e et/ou 8e groupe secondaire de la classification périodique des éléments.

2. Utilisation selon la revendication 1, caractérisée en ce que le métal modifiant est sélectionné dans le groupe comprenant le fer, le chrome, le cuivre, le molybdène, le tantale, le tungstène, le vanadium, le titane, le niobium, le rhénium, le ruthénium, le zirconium et le hafnium.

3. Utilisation selon la revendication 1, caractérisée en ce qu'on exploite la chaleur réactionnelle pour former de la vapeur avec des pressions d'émission situées entre 5 et 30 bar.